**Europäisches Patentamt**

**European Patent Office**

**Office européen des brevets**

(19)

(11) Veröffentlichungsnummer: **0 008 417**
**A1**

(12)

# EUROPÄISCHE PATENTANMELDUNG

(21) Anmeldenummer: **79102894.7**

(22) Anmeldetag: **10.08.79**

(51) Int. Cl.³: **A 61 M 5/14**
**G 05 D 7/06**

(30) Priorität: **22.08.78 DE 2836604**

(43) Veröffentlichungstag der Anmeldung:
**05.03.80 Patentblatt 80/5**

(84) Benannte Vertragsstaaten:
**BE CH DE FR GB IT NL SE**

(71) Anmelder: **BAYER Aktiengesellschaft**
**Zentralbereich Patente,Marken und Lizenzen Bayerwerk**
**D-5090 Leverkusen 1(DE)**

(72) Erfinder: **Brauner, Dieter**
**Weyer-Strasse 276**
**D-5650 Solingen 19(DE)**

(72) Erfinder: **Reuter, Rolf, Dipl.-Ing.**
**Am Gierlichshof 26**
**D-5090 Leverkusen 3(DE)**

(54) **Vorrichtung zum genauen Dosieren von Flüssigkeiten.**

(57) Die Dosiervorrichtung besteht im Prinzip aus einem abgeschlossenen Vorratsbehälter (6) für die zu dosierende Flüssigkeit (5), der an seinem unteren Ende eine Entnahmeleitung und eine Belüftungsleitung (1) aufweist. Die Dosierrate wird über den Luftstrom, der durch die Belüftungsleitung in die Flüssigkeit einperlen kann, eingestellt.

Das Ventil (3, 10) besteht vorzugsweise aus einem reinen Absperrorgan (offen oder geschlossen), das elektromagnetisch oder pneumatisch gesteuert ist und von einem Generator (4) mit wählbarer Puls-frequenz und Pulsdauer gespeist wird.

FIG 1

EP 0 008 417 A1

BAYER AKTIENGESELLSCHAFT     5090 Leverkusen, Bayerwerk
Zentralbereich
Patente, Marken und Lizenzen

Vorrichtung zum genauen Dosieren von Flüssigkeiten

Die Erfindung betrifft eine Vorrichtung zum genauen Dosieren von Flüssigkeiten. Sie besteht im Prinzip aus einem abgeschlossenen Vorratsbehälter mit einer Entnahmeleitung, durch die die Flüssigkeit unter Schwerkrafteinwirkung zum Ort der Dosierung abfließt. Die Vorrichtung eignet sich insbesondere zur Verabreichung von Infusionslösungen. In diesem Falle führt die Entnahmeleitung direkt zum Patienten.

Bekanntlich werden wegen der größeren Sicherheit und Genauigkeit, der Ersparung von Personalkosten und der besseren Überwachungsmöglichkeit Infusionen an Kranken neuerdings unter anderem über Infusionsregelgeräte vorgenommen. Dabei wird versucht, über die Zeitdauer einer Infusion eine konstante Tropfenrate, die meist lichtoptisch innerhalb einer Tropfkammer gezählt wird, zu erreichen. Das wird durch handelsübliche und in der Patentliteratur unter den Nummern

Le A 18 784

- 2 -

DE-OS 15 41 363, DE-PS 19 62 191, DE-OS 20 40 714, DE-OS 22 24 926, DE-OS 22 45 274, DE-OS 24 19 520, DE-OS 24 20 966 und DE-OS 24 52 117 beschriebene Geräte dadurch bewerkstelligt, daß der Durchfluß durch den Schlauch, der von der Tropfkammer zum Patienten führt, durch eine motorisch betätigte Drosselstelle mehr oder weniger stark gebremst wird, so daß durch eine elektrische Rückkopplungsschaltung immer eine konstante Tropfenrate eingestellt wird. Der Betrieb einer Tropfkammer war früher und ist bis jetzt noch immer die einfachste Methode zur Einstellung eines gewünschten Mengenstromes.

Diese Geräte haben den Nachteil, daß bei den verschiedenen, gewünschten Tropfenraten (z.B. 30 Tr/min) nicht von vornherein die zugehörigen Mengenströme $\dot{V}$ [ml/h] bekannt sind. Es besteht nämlich keine lineare Korrelation zwischen der Tropfenrate und der ausgetropften Masse. Je größer die Tropfenzahl pro Zeiteinheit wird, desto mehr steigt überproportional der Mengenstrom, so daß schließlich bis zu 30 % Fehler gegenüber einer gewünschten Menge auftreten können. Dieser Nachteil wird noch dadurch verstärkt, daß das Tropfenvolumen auch für eine bestimmte Tropfenrate nicht allgemein angegeben werden kann. Die übliche Annahme von 20 Tr/ml ist deshalb falsch, weil das Tropfenvolumen sowohl von der Tropfenrate wie auch von der Geometrie der Tropfenbildungsöffnung, der Temperatur, der Zähigkeit, der Oberflächenspannung, der Dichte und verschiedenen anderen Einflußgrößen abhängt. Schließlich besteht ein erheblicher Nachteil dieser Geräte auch darin, daß zur Regelung ein Flüssigkeitsdrosselorgan, im einfachsten Falle eine Schlauchquetsche betätigt werden muß, das erhebliche Stellkräfte bzw. einen entsprechend schweren Antrieb, verbunden mit hohem Energieverbrauch, benötigt. Bei der Tropfenkammer bestehen Nachteile darin, daß die Tropfenbildungsöffnung sich im Laufe der Zeit verändert (z.B. durch Ansätze) und daß die gesamte Kammer als Steril-

Le A 18 784

teil ausgeführt werden muß. Diese Nachteile konnten durch die bisher auf dem Markt erschienenen Infusionsregelgeräte nicht beseitigt werden.

Die weiterhin bekannt gewordenen Vorrichtungen, die die Sekundärluftzufuhr in ein formstabiles Infusionsvorratsgefäß regulieren, erfüllen ebenfalls nicht die heutigen Anforderungen an volumetrische Genauigkeit der Verabreichung von Infusionen.

Beschrieben werden z.B. handbetätigte Drosselstellen in der Sekundärluftzuleitung (DE-AS 14 91 708, DE-AS 20 22 107) oder ein selbsttätiges Kugelventil in der Sekundärluftsausleitung (DE-PS 19 15 204). Mit solchen Vorrichtungen erreicht man noch nicht einmal eine Konstanz der Tropfenrate über einen Infusionszyklus; diese Geräte dienen lediglich als Ersatz für die sonst verwendete, jedoch auch unzureichende, Rollenquetsche. Als gefährlich kann sogar die in der DE-OS 26 06 914 beschriebene Sekundärluftzufuhr über einen Kompressor angesehen werden, da hierbei im Fehlerfall ein Druckpolster im Vorratsgefäß aufgebaut werden kann. Auch mit einer Handdruckpumpe zur Belüftung des Vorratsgefäßes (DE-OS 16 16 208) kann die Genauigkeit nicht gesteigert werden.

In allen Fällen müssen die unvorteilhaften Tropfenkammern notwendigerweise erhalten bleiben.

Der Erfindung lag nun die Aufgabe zugrunde, eine Vorrichtung zu schaffen, die mit einfachsten Mitteln, möglichst ohne eine Tropfenkammer zwingend zu benötigen und ohne großen Energieverbrauch eine volumetrische Infusion zu verabreichen gestatten und über einen Infusionszyklus (Volumen des Vorratsgefäßes) eine hohe Konstanz in der Strömungsrate bewirkt. Insbesondere sollte dazu keine Nachstellung durch Überwachungs-

Le A 18 784

- 4 -

personal erforderlich sein.

Diese Aufgabe wird erfindungsgemäß dadurch gelöst, daß in den Vorratsbehälter eine Belüftungsleiter mündet, in der sich ein Ventil befindet, das eine gesteuerte Zugabe von Sekundärluft in den rückwärtigen Teil des Vorratsbehälters ermöglicht. Durch das Ventil wird im Vorratsbehälter eine definierte Luftmenge zugeführt.

Vorzugsweise endet die Belüftungsleitung unterhalb des Flüssigkeitsniveaus im Vorratsbehälter.

Besonders bewährt hat sich eine Ausführungsform der Erfindung, bei der das Ventil elektromagnetisch oder pneumatisch gesteuert ist und von einem Generator mit wählbarer Pulsfrequenz und Pulsdauer gespeist wird. Die Zugabe der Sekundärluft erfolgt in diesem Falle periodisch. Dadurch wird erreicht, daß eine volumengleiche Menge Infusionsflüssigkeit aus dem Vorratsgefäß ausfließen kann.

In jedem geschlossenen Gefäß, dessen Ausflußöffnung an der untersten Stelle liegt, stellt sich im über der Flüssigkeit befindlichen Luftraum ein Unterdruck ein, der verhindert, daß das Gefäß leerläuft, wenn nicht das gleiche Volumen an Luft einströmen kann. Am Ende der Infusionsleitung wird am Patienten wieder ein gewünschter Überdruck dadurch eingestellt werden, daß das Infusionsvorratsgefäß die entsprechende geodätische Höhe oberhalb der Infusionsstelle am Patienten hat. Die Größe des Unterdruckes im Vorratsgefäß wird durch die geodätische Höhe des Flüssigkeitsspiegels und den Überdruck am Patienten bestimmt. Der Unterdruck ändert sich im Laufe eines Infusionszyklus entsprechend dem fallenden Flüssigkeitsspiegel und zwar bei konstant bleibendem Gegen-

Le A 18 784

druck von Seiten des Patienten um ca. 20 mbar. Um diesen Betrag wird der absolute Druck im Sekundärluftraum des Vorratsgefäßes größer, unabhängig, ob mit oder ohne Tropfkammer gearbeitet wird. Damit geht der Unterdruck bei sich entleerendem Gefäß in Richtung Null zurück, wozu ca. 500 ml Luft eingeströmt sein müssen.

Im genannten Bereich des Unterdruckes kann man nun die Volumenverfälschung durch Kompressibilität im allgemeinen noch vernachlässigen. (Das Luftvolumen ändert sich um 0,1 % bei einer Druckänderung um 1 mbar; zum Vergleich: eine Temperaturänderung um 1 $^\circ$K verändert das Luftvolumen oder den Luftdruck um ca. 0,3 %.) Im speziellen Fall des erfindungsgemäßen Verfahrens der Luftzufuhr zu Infusionsflaschen können also Dosierfehler von max. 2 % entstehen, d. h., die Infusionsrate $\dot{V}$ [ml/h] geht im Laufe eines Infusionszyklus um 2 % zurück. Dies ist auf das zu Beginn der Infusion beim Aufperlen durch den Flüssigkeitsvorrat geringfügig größer werdende Blasenvolumen zurückzuführen.

Somit ist der Fehler erheblich geringer als bei herkömmlichen Infusionshilfsmitteln und im Rahmen der Fehler, die durch mögliche Temperaturschwankungen von ± 3 $^\circ$K in jedem Falle auch entstehen.

Entgegen der vorherrschenden Meinung kann eine volumetrische Flüssigkeitsdosierung erreicht werden, indem die Sekundärluftmenge gesteuert wird. Überraschenderweise kann dabei auf eine Tropfkammer zur Kontrolle des Mengenflusses verzichtet werden, was sich als zusätzlicher Vorteil erweist. Mit diesem Verfahren kann eine Infusion solange durchgeführt werden, wie Unterdruck oberhalb des Flüssigkeitsspiegels herrscht.

Le A 18 784

- 6 -

Die Infusion ist automatisch beendet, wenn im Sekundärluftvolumen Atmosphärendruck herrscht.

Die Sekundärluftmengensteuerung geschieht erfindungsgemäß
dadurch, daß über ein Relais die Luftansaugöffnung des Infusionsbesteckes periodisch verschlossen und geöffnet wird,
so daß in der Zeiteinheit eine bestimmte Menge Luft in Form
von Einzelblasen durch die Flüssigkeit in den Sekundärluftraum einperlt. Die Größe der pro Relaisimpuls gebildeten
Blase muß durch die Impulsbreite (Öffnungszeit des Relais),
vorzugsweise auch durch ein in die Luftansaugöffnung oder
in die Schlauchleitung von dieser Öffnung bis zum Relais
eingebautes Bakterienfilter oder eine Kapillare eingestellt
werden. Für jede einperlende Luftblase kann ein gleiches
Volumen Flüssigkeit das Vorratsgefäß verlassen, wobei sich
in einer Tropfkammer eine beliebige, für das erfindungsgemäße Verfahren unbedeutende Tropfenfrequenz einstellen muß,
wenn man nicht vorzieht ganz ohne Tropfkammer zu arbeiten.

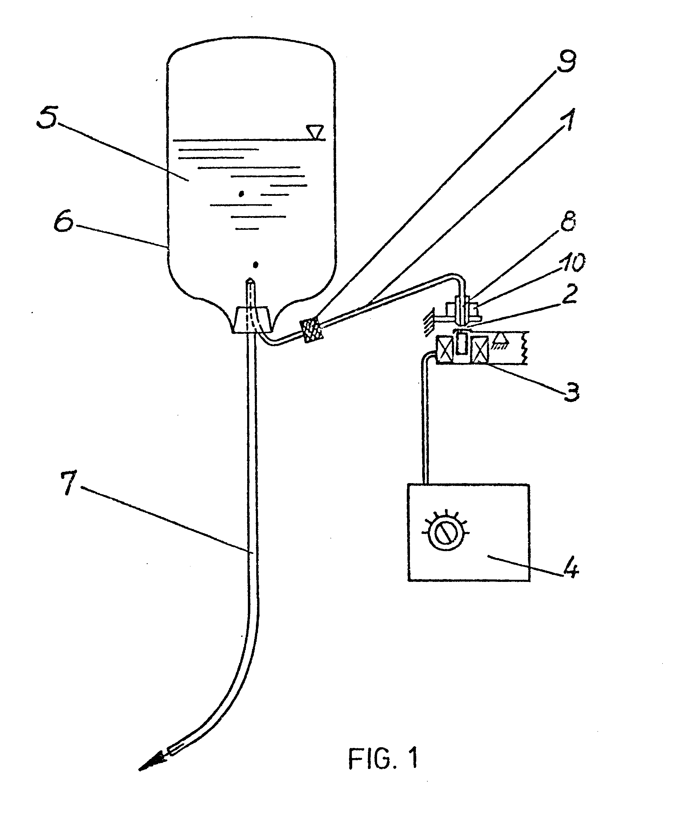

Im folgenden ist ein Ausführungsbeispiel der Erfindung unter
Bezugnahme auf die Zeichnung, Bild 1, und die Zahlensymbole
näher erläutert. Die erfindungsgemäße Vorrichtung zum Verfahren besteht aus der verlängerten Belüftungsleitung in
Form eines Schlauches 1, der Ansaugöffnung für Sekundärluft
2 und dem Relais 3, das vom Impulsgerät 4 beaufschlagt wird.
Infusionsflüssigkeit 5 gelangt aus der Vorratsflasche 6 durch
eine Ausflußöffnung in den Schlauch 7 und fließt dem Patienten zu. Die Luftzufuhr erfolgt von der Eintrittsstelle 2 aus
vorzugsweise durch eine Kapillare 8 von z. B. 0,2 - 0,5 mm
Durchmesser und vorzugsweise 20 - 40 mm Länge und/oder durch
ein Filter 9. Die Dichtkräfte an der Stelle 2 wie auch die
Öffnungszeit können über eine Verstelleinrichtung 10 eingestellt werden. Wird die Öffnungsstelle 2 kurzzeitig, z.B.
durch einen Stromimpuls von 10 ms Dauer, freigegeben, so

Le A 18 784

wird ein geringes Luftvolumen in die Stelle 2 eingesaugt und perlt als Blase in den Luftraum oberhalb der Infusionslösung. Bei Vorhandensein einer Tropfkammer wird diese als zusätzliche Möglichkeit für individuelle optische Kontrolle benutzt werden können. Im Normalfall erkennt man jedoch an den aufperlenden Blasen im Vorratsgefäß die einwandfreie Funktion einer Infusion nach dem beschriebenen Verfahren.

Mit einer Vorrichtung wie hier beschrieben, wurde das Ausfließen bei Impulszahlen zwischen 5 und 200 I/min geprüft; wir erhielten folgenden Zusammenhang zwischen Impuls und Infusionsrate:

$$
\begin{aligned}
5 \text{ Imp/min} &\,\hat{=}\,\ 18,8 \text{ ml/h} \\
10 \text{ Imp/min} &\,\hat{=}\,\ 35,5 \text{ ml/h} \\
20 \text{ Imp/min} &\,\hat{=}\,\ 78,4 \text{ ml/h} \\
50 \text{ Imp/min} &\,\hat{=}\, 191,5 \text{ ml/h} \\
100 \text{ Imp/min} &\,\hat{=}\, 379 \quad \text{ml/h} \\
150 \text{ Imp/min} &\,\hat{=}\, 563 \quad \text{ml/h} \\
200 \text{ Imp/min} &\,\hat{=}\, 763 \quad \text{ml/h.}
\end{aligned}
$$

Le A 18 784

- 8 -

Patentansprüche:

1. Vorrichtung zum genauen Dosieren von Flüssigkeiten, insbesondere zur Verabreichung von Infusionslösungen, bestehend aus einem abgeschlossenen Vorratsbehälter mit einer Entnahmeleitung, dadurch gekennzeichnet, daß in den Vorratsbehälter (6) eine Belüftungsleitung (1) mit einem Ventil (3,10) mündet, das eine gesteuerte Zugabe von Sekundärluft in den rückwärtigen Teil des Vorratsbehälters ermöglicht.

2. Vorrichtung nach Anspruch 1, dadurch gekennzeichnet, daß die Belüftungsleitung (1) unterhalb des Flüssigkeitsniveaus im Vorratsbehälter (6) endet.

3. Vorrichtung nach Anspruch 1 bis 2, dadurch gekennzeichnet, daß das Ventil (3,10) elektromagnetisch oder pneumatisch gesteuert und von einem Generator (4) mit wählbarer Pulsfrequenz und Pulsdauer gespeist wird.

4. Vorrichtung nach Anspruch 1 bis 3, dadurch gekennzeichnet, daß in der Belüftungsleitung (1) eine Kapillare (8) angeordnet ist.

5. Vorrichtung nach Anspruch 1 bis 4, dadurch gekennzeichnet, daß in der Belüftungsleitung (1) ein Bakterienfilter (9) angeordnet ist.

Le A 18 784

FIG. 1

0008417

0008417

Europäisches
Patentamt

**EUROPÄISCHER RECHERCHENBERICHT**

Nummer der Anmeldung

EP 79 10 2894

| | EINSCHLÄGIGE DOKUMENTE | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | DE - A - 1 810 802 (METZ) <br> * Abbildung 7; Seite 1, 1.Absatz; Seite 7, 3.Absatz; Seite 8, 1.Absatz * <br><br> ---- | 1,4 | A 61 M 5/14 <br> G 05 D 7/06 |

**RECHERCHIERTE SACHGEBIETE (Int. Cl. 3)**

A 61 M
G 01 F
G 05 D

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie. übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 21-11-1979 | NOESEN |

EPA form 1503.1 06.78